(19) Europäisches Patentamt
European Patent Office
Office européen des brevets

(11) **EP 1 872 788 B1**

(12) **EUROPEAN PATENT SPECIFICATION**

(45) Date of publication and mention
of the grant of the patent:
**22.05.2013  Bulletin 2013/21**

(21) Application number: **06731419.5**

(22) Date of filing: **07.04.2006**

(51) Int Cl.:
*A61K 33/18* (2006.01)     *A61K 9/06* (2006.01)
*A61K 9/08* (2006.01)      *A61K 9/10* (2006.01)
*A61K 9/70* (2006.01)      *A61K 47/06* (2006.01)
*A61K 47/32* (2006.01)     *A61K 47/44* (2006.01)
*A61P 17/02* (2006.01)

(86) International application number:
**PCT/JP2006/307472**

(87) International publication number:
**WO 2006/109734 (19.10.2006 Gazette 2006/42)**

(54) **EXTERNAL AGENT FOR TREATING WOUNDS**

TOPISCHES MITTEL ZUR BEHANDLUNG VON WUNDEN

AGENT EXTERNE POUR TRAITER DES PLAIES

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **08.04.2005  JP 2005112174**

(43) Date of publication of application:
**02.01.2008  Bulletin 2008/01**

(73) Proprietors:
• **Mie University
Tsu-shi, Mie 514-0102 (JP)**
• **Maruishi Pharmaceutical Co., Ltd.
Osaka 538-0042 (JP)**

(72) Inventors:
• **MIZUTANI, Hitoshi
c/o Mie University
Tsu-shi, Mie 5148507 (JP)**
• **MIYAMOTO, Yoshiaki
Tsurumi-ku
Osaka 538-0042 (JP)**
• **NISHIKAWA, Atsushi
Tsurumi-ku
Osaka 538-0042 (JP)**

(74) Representative: **Teipel, Stephan et al
Lederer & Keller
Patentanwälte
Unsöldstrasse 2
80538 München (DE)**

(56) References cited:
**EP-A- 1 449 521     EP-A2- 0 185 490
WO-A-01/47534     WO-A1-2004/011032
AU-B2- 506 419     JP-A- 10 001 443
JP-A- 11 049 672     US-A- 5 128 125**

Remarks:
The file contains technical information submitted after
the application was filed and not included in this
specification

**Description**

FIELD OF THE INVENTION

This invention is related to a drug for external use for treatment of diseases accompanied by skin injuries, such as erosion, decubitus, skin ulcer, and burns.

DESCRIPTION OF THE BACKGROUND ART

Skin tissue and mucosa are often injured by various causes.
These skin tissue injuries and ulcer are generally called 'wounds'. For example, erosion, cut, abrasion, burns, decubitus, and skin ulcer are included.
These wounds are painful, and may cause infection. Further, keloid-like lesions are caused and scars may remain when problems occur in the wound healing process. Thus, it is preferable that wounds are completely healed in the early stage.
Particularly, decubitus and skin ulcer are likely to occur in not only bed-ridden elderly persons but also persons with disabilities, the poorly nourished, and weak recovering abilities. Decubitus and skin ulcer have been a social problem because these persist for a prolonged period.
Decubitus represents a pathology in which skin compression inhibits local blood flow, resulting in injury of the skin tissue due to circulatory failure. Mild decubitus is a sore on the skin surface, but skin ulcers are formed in the subcutaneous tissue, bone, and ligament in severe decubitus.
Severe decubitus is very intractable, and it may lead to death due to bacterial infection.
Development of various drugs for wound treatment to promote wound healing, particularly those for external use, has recently been progressing.
Examples include fibrinolytic enzymes, such as fibrinolysin, deoxyribonuclease, streptokinase, and streptodornase, necrotomy tissue agents containing lysozyme chloride, antimicrobial agents containing gentamicin sulfate, sulfadiazine silver, bacitracin, and fradiomycin sulfate, incarnant agents containing trafermin, bucladesine sodium, tretinoin tocoferil (tocoretinate), alprostadil alfadex, solcoseryl (extract from hemolysed blood of young cattle), and alcloxa, iodine preparations containing white soft sugar, povidone iodine, and iodine, and preparations containing bendazac, dimethyl isopropylazulene (guaiazulene), and epinephrine as active ingredients.
Especially, white soft sugar is known to exhibit a wound healing effect, and povidone iodine has bactericidal activity (due to iodine). Wound treatment drugs for external use consisting of a mixture of white soft sugar and povidone iodine have been used for treatment of wounds, such as decubitus and skin ulcer, as preparations that exhibit both wound healing and bactericidal effects.
Currently, white soft sugar/povidone iodine combined drugs with improved pharmaceutical stability and homogeneity are commercially available and widely used for treatment of wounds including decubitus in clinical practice.
Various wound treatment drugs for external use including white soft sugar/povidone iodine-combined-drugs and various wound protective materials as medical devices are supplied for medical practice, and wound treatment means are progressing. However, decubitus and skin ulcer are still intractable diseases, and promotion of healing of decubitus will become an important issue as society ages.
Drugs for external use targeting promotion of wound healing generally contain white soft sugar at a high concentration to increase the effect (patent document 1). However, these drugs for external use do not promote wound healing. Futher, these drugs for external use induce a negative reaction such as cicatrization.
Many of these previous drugs contain water-soluble bases, prioritizing drying and cleanliness of the wound surface, but protection of the wound surface is not considered.
Moreover, iodine preparations described above are accumulated on the wound surface and surrounding skin. Residual iodine is a concern of adverse events, such as skin staining (so called iodine burns) and irritation by iodine preparation themselves. These drugs have many other problems, such as contraindication for patients with specific diseases.
EP-A-1 449 521 discloses the use of PVP-iodine liposomes for treatment of atopic dermatitis.
JP 11 049 672 A relates to a plaster for wound treatment including a sugar alcohol as an active ingredient.
EP-A-0 185 490 relates to a topical, antibacterial cream containing an antibacterial agent and a base, the base having a 20 to 50 % (w/w) content of hydrocarbon and polyol moisturising components.
JP 10 001 443 A discloses a preparation for external use which is obtained by blending 0.001-50wt.% sterilizing disinfectant for exodermis such as iodine, an iodine tincture, iodoform, boric acid, phenol, a saponated cresol solution, benzalkonium chloride or mercurochrome with 0.2-80wt.% vitamin E and/or 0.5-80wt.% squalane and further one or more percutaneous absorbefacients such as an N-acylsarcosine (salt), a higher fatty acid ester which is a reactional product of a 10-18C higher fatty acid with a 1-20C alcohol, a 2-10C dicarboxylic acid (salt), a hydrocarboxylic acid ester that is a reactional product of a 3-6C hydrocarboxylic acid with the 1-20C alcohol and a fatty acid monoethanolamide.
Accordingly, development of a new low-toxic drug for external use that prevents cicatrization and reduces iodine accumulation on the wound surface with sufficiently promoting healing has been strongly needed for treatment of intractable diseases, such as decubitus and skin ulcer.

[Patent document 1]

Publication of patent application No. 2000-38342

DISCLOSURE OF INVENTION

PROBLEMS OF THE INVENTION AIMS TO SOLVE

The objective of this invention is to provide a new low-toxic drug for external use for intractable diseases, such as decubitus and skin ulcer, that prevents cicatrization and reduces iodine accumulation on the wound surface with sufficiently promoting healing, in view of the above problems.

MEANS TO SOLVE PROBLEMS

The invention is described in the claims.

EFFECT OF THE INVENTION

A wound treatment drug for external use of this invention prevents infection through wound surface by the bactericidal action, and maintains appropriate moisture of the wound surface by preventing excessive drying of the wound surface.
Subsequently, the drug of this invention enhances the wound treatment effect, prevents cicatrization, and reduces iodine accumulation on the wound surface.

DESCRIPTION OF THE PREFERRED EMBODIMENTS

As a result of a keen study, the inventors found that a wound treatment drug for external use composed of a combination of iodine with a bactericidal effect and oily bases with a moisturizing effect substantially containing no white soft sugar, which has a wound healing effect, is useful for treatment of intractable diseases such as decubitus and skin ulcer, and completed this invention.
A wound treatment drug for external use of this invention prevents infection through wound surfaces by sufficient bactericidal action of iodine, and at the same time, a moisturizing effect of oily bases enables maintenance of appropriate moisture of the wound surface, which result in promotion of healing, prevention of cicatrization, and reduction of residual iodine on the wound surface.
The inventors also discovered that the absence of white soft sugar, which is contained at a high concentration in the current technique to promote wound healing, prevents excessive drying of wound surfaces. They also discovered that the drug promotes healing without inducing cicatrization.
Thus, the substantial absence of white soft sugar is a characteristic of the wound treatment drug for external use of this invention. This characteristic avoids negative effects, such as reduction of the healing effect due to excessive drying of wound surfaces and induction of cicatrization.
The specific point of this invention is that the preparations contain oily bases, such as petrolatum and gelated hydrocarbon, enables to maintain appropriate moisture of wound surfaces. That results in promotion of healing, prevention of cicatrization, and reduction of residual iodine on the wound surface.
Iodine and its preparations have been used as disinfectants for a long time, and oily bases, such as petrolatum and gelated hydrocarbon, are widely used as skin protective agents and bases of various preparations, and their safety has been confirmed.
However, the above combination has not been applied as active ingredients of therapeutic drugs for diseases accompanied by skin injuries, such as erosion, decubitus, skin ulcer, and burns.
Embodiments for carrying out this invention to exert these effects are described in detail below.
Any oily base generally used for ointments can be used for this invention, but petrolatum and gelated hydrocarbon are preferably used.
Oily bases mixed with petrolatum and gelated hydrocarbon may also be used.
'Petrolatum' used for this invention may be white, yellow, and hydrophilic petrolatum. Preferably, 'white petrolatum' and 'hydrophilic petrolatum' are used.
'White petrolatum' is prepared by decolorization and purification of a mixture of petrolic hydrocarbons. And products listed in the Japanese Pharmacopoeia may be used as white petrolatum.

'White petrolatum', which is generally available, includes 'ointment base Japanese Pharmacopoeia white petrolatum 500 g' of Maruishi Pharmaceutical Co., Ltd.

'Hydrophilic petrolatum' is a mixture of white petrolatum, white beeswax, stearyl alcohol, and cholesterol. And products listed in Japanese Pharmacopoeia may be used as hydrophilic petrolatum.

'Hydrophilic petrolatum' , which is generally available, includes 'ointment base Japanese Pharmacopoeia hydrophilic Petrolatum 500 g' of Maruishi Pharmaceutical Co., Ltd.

'Gelated hydrocarbon' used for this invention includes a mixture of liquid paraffin and polyethylene resin. 'Gelated hydrocarbon', which is generally available, includes 'Plastibase' (registered trademark) of Taisho Pharmaceutical Co., Ltd.

The content of oily bases, such as petrolatum and gelated hydrocarbon, in a wound treatment drug for external use of this invention is 50-99 weight%, preferably, 70-97 weight%, and more preferably, 85-95 weight%.

The moisturizing effect is not sufficiently exerted when the content is less than 50 weight%, and preparation is difficult when the content is higher than 99 weight%. Thus, these contents are inappropriate.

'Iodine' used in this invention is used as complexes with 1-vinyl-2-pyrrolidone polymer (povidone iodine) and surfactant.

The content of 'iodine' in a wound treatment drug for external use is 0.01-5 weight%, preferably 0.05-2 weight%, and more preferably 0.1-0.9 weight%.

The bactericidal effect is not sufficiently exerted when the content is less than 0.01 weight%. Iodine preparations accumulate on the wound surface and surrounding skin when the content is higher than 5 weight%. And the residual iodine may exhibit adverse effects, such as skin staining (so called iodine burns) and irritation by iodine preparations themselves. Thus, these contents are inappropriate.

As a characteristic of a wound treatment drug for external use of this invention, the drug contains substantially no white soft sugar, which is considered to have wound healing action.

Such compositions avoid negative effects: reduction in the healing effect due to excessive drying of the wound surface and induction of cicatrization.

White soft sugar described here includes hygroscopic sugar group, such as purified sugar, for example, Japanese Pharmacopoeia listed white soft sugar and sucrose.

The sugar group induces cicatrization of the wound surface.

Current wound treatment drugs for external use contain white soft sugar at a high concentration (50-90 weight%), and the wound healing effect of white soft sugar has been considered to promote wound healing. However, these drugs have not sufficiently exhibited the expected effect, but induced cicatrization.

The inventors discovered that the above negative effects due to excessive drying of wound surfaces can be prevented by containing substantially no scrose at a high concentration, and subsequently developed a wound treatment drug for external use of this invention characterized in containing substantially no white soft sugar.

'Containing substantially no white soft sugar' means containing no white soft sugar or containing white soft sugar at a level which does not exert pharmacological activities, such as wound healing.

The level which does not exert pharmacological activities may be any content lower than the minimum combined amount required for exertion of pharmacological activities of white soft sugar, but less than 20 weight% is preferred.

A wound treatment drug for external use of this invention contains an oily base and iodine preparation described above as essential ingredients, and substantially no white soft sugar. The drug prevents infection through wound surfaces due to a sufficient bactericidal effect of iodine therein, and enables to maintain appropriate moisture of wound surfaces due to moisturizing effects of oily bases therein. As a result, the wound treatment effect may be enhanced.

Furthermore, the drug enhances wound healing, prevents cicatrization, and reduces iodine accumulation on wound surfaces by maintaining moist wound surfaces.

At the same time, the drug does not exhibit negative effects, such as delay in healing and induction of cicatrization, because of containing substantially no white soft sugar which may cause excessive drying of wound surfaces.

A wound treatment drug of this invention can be formulated in known dosage forms for external use using the above active ingredients and pharmacologically acceptable drug carriers.

Dosage forms are preferably paste, ointment, cream, liquid, gel, adhesive, cataplasm, and patch.

A wound treatment drug for external use of this invention can be prepared and manufactured corresponding to the above dosage forms in which the above iodine components are mixed and homogenized by the standard method using conventional pharmaceutical techniques.

For example, the drug is prepared by slowly adding various bases and specific components to povidone iodine and white petrolatum, kneading them and homogenizing them.

In addition to the above active ingredients, components below are included as examples of base components used in the drug of this invention.

As for lipid, for example, middle-chain triglyceride, synthetic oil, such as hard fat, plant oils, such as olive oil, soy bean oil, canola oil, peanut oil, safflower oil, rice bran oil, sesame oil, camellia oil, corn oil, cotton seed oil, and coconut oil, animal oils, such as lard and beef tallow, and solidified oils of these are used.

As for wax, for example, natural waxes, such as lanolin, yellow beeswax, carnauba wax, and spermaceti, and mineral waxes, such as montan wax, and synthetic wax, are used.

As for hydrocarbon, for example, paraffin, liquid paraffin, microcrystalline wax, squalene, polyethylene powder, and gelated hydrocarbon are used.

As for higher fatty acid, for example, stearic acid, behenic acid, palmitic acid, and oleic acid are used.

As for higher alcohol, for example, cetanol, stearyl alcohol, oleyl alcohol, and cholesterol are used. For polyhydric alcohol, for example, propylene glycol, polyethylene glycol, glycerin, and 1,3-butylene glycol are used.

As for synthetic and natural macromolecules, for example, carrageena, starch, dextrin, dextrin polymer (cadexomer), polyoxyethylene polyoxypropylene glycol (poloxamer), tragacanth, Arabic gum, locust bean gum, pectin, gelatin, xanthan gum, pullulan, alginate, hydroxypropylcellulose, sodium carboxymethylcellulose, polyacrylate, polyvinyl alcohol, polyvinyl pyrrolidone, and carboxyvinyl polymer are used.

As for surfactant, for example, alkyl sulfate, polyoxyethylene alkylether phosphate, glycerin fatty acid ester, polyglycerin fatty acid ester, sorbitan fatty acid ester, polyoxyethylene sorbitan fatty acid ester, polyxyethylene solidified castor oil, polyethylene glycol fatty acid ester, polyoxyethylene polyoxypropylene glycol, polyoxyethylene alkyl ether, and sucrose fatty acid ester are used.

As for lower alcohol, for example, ethanol and isopropyl alcohol are used.

As for ketone, for example, acetone and methylethyl ketone are used.

As for powder, for example, kaolin, talc, zinc oxide, titanium oxide, magnesium stearate, anhydrous silicic acid, and starch are used.

As for cellulose derivative, for example, methylcellulose, sodium carboxymethylcellulose, and hydroxypropylcellulose are used.

As for inorganic salt, for example, potassium iodide, sodium iodide, sodium sulfate, sodium hydrogen carbonate, sodium chloride, calcium nitrate, potassium nitrate, sodium nitrate, aluminum sulfate, sodium polyphosphate, ammonium chloride, iron sulfate, sodium phosphate, magnesium sulfate, sodium thiosulfate, sodium sesquicarbonate, sodium sulfate, borax, calcium oxide, magnesium carbonate, and potassium chloride are used.

The relationship between each dosage form and additives are more concretely described below.

As for paste, the drug can be used as <u>oily paste</u>. As for the base of paste, for example, lipids, waxes, and hydrocarbons are used.

As for ointment, for example, lipids, polyhydric alcohols, and hydrocarbons are used as the base.

As for cream, for example, surfactants, higher alcohols, higher fatty acids, hydrocarbons, polyhydric alcohols, and water (purified water) are used as the base.

As for liquid and gel, for example, water (purified water), lower alcohols, ketones, lipids, polyhydric alcohols, surfactants, hydrocarbons, and synthetic and natural macromolecules are used as the base.

In addition, alkali metal hydroxides, such as sodium hydroxide, may be used as a pH adjuster, as needed in the drug of this invention. Antiseptics/preservatives may also be combined, for example, alkali metal benzoates, such as sodium benzoate, p-hydroxybenzoate esters, and sorbic acid.

Furthermore, antioxidants, for example, tocopherol, dibutyl hydroxytoluene, and butyl hydroxyanisole, may be used, as needed.

The wound treatment drug for external use of this invention prepared above is formulated as W/O emulsion.

The drug for skin application of this invention may be directly applied to affected regions. Furthermore, for example, adhesives, such as cataplasms and plasters prepared by spreading the drug on elastic and nowoven and plastic sheets, may be applied to affected regions.

The wound treatment drug for external use of this invention prepared above is safe for skin and results in comfortable use. They are also clinically superior and extremely effective for promoting wound healing.

Thus, the drug exhibits a sufficient effect on healing of wounds: erosion, cut, abrasion, burns, decubitus, and skin ulcer.

Examples

The invention is explained with examples below, but not limited to the examples.

Combined amounts are presented as weight%, not otherwise specified.

[Example 1] Effect on the wound healing process in rats

A wound treatment drug for external use of this invention for skin application (ointments) was prepared using the following formula and method.

Drugs used for the formula example are shown in 1) and 2).

1) Hydrophilic petrolatum listed in Japanese Pharmacopoeia (Maruishi Pharmaceutical Co., Ltd.)
The preparation contained 8 g of white beeswax, 3 g of stearyl alcohol, 3 g of cholesterol, an appropriate amount of white petrolatum, and dibutyl hydroxytoluene as an additive per 100 g of the above hydrophilic petrolatum. This preparation is used as an ointment base and skin protector.

2) Povidone iodine (Sigma Co.)

3) Polyethylene glycol 400 (Wako Pure Chemical Industries Ltd.)

(Formula 1 (100 g))
Hydrophilic petrolatum: 90.0 g
Povidone iodine: 3.0 g
Polyethylene glycol 400: 3.5 g
Purified water: 3.5 g
(Control drug)
Formula of U-PASTA KOWA (100 g)
Sucrose: 70.0 g
Povidone iodine: 3.0 g
Other additives, such as macrogol
(Production method)

The substances were mixed at the above ratio and kneaded to a semi-solid state.

(Promotion of wound healing in rats)

[Experimental methods]

S1c:SD rats (male, 6 weeks of age) were used. Under pentobarbital anesthesia, the rats were restrained on a recumbent position. The dorsal skin was slightly stretched and punched using a punch for leather (diameter: 1.8 cm). Symmetric full-thickness defective wounds were made by this procedure. Formula 1 and a commercial drug for treatment of decubitus containing 3% povidone iodine and 70% white soft sugar, U-PASTA KOWA (Kowa Co., Ltd.), were individually applied once a day at 150 mg to the wounds for 7 days.

In a control group, wounds were made and mock treatment was applied to the wounds for 7 days.

The dorsal region was photographed using a digital camera 3 times a week, and the wound areas were measured using imaging analysis software, NIH Image (the National Institutes of Health of the U.S.A.).

The wound area regards the mean of the right and left wound areas of each rat, and the mean and standard deviation of 5 rats were presented.

The dorsal skin including the wound surfaces was excised after application for 7 days in each group, and fixed in 10% neutral buffered formalin solution. Paraffin sections were prepared, stained with hematoxylin-eosin, and observed including pathological observation.

[Results]

Based on Table 1 <Reduction of wound area and healing rate> shown below, the wound area was reduced, and the healing rate was about 64% in the Formula 1 treatment group. In contrast, no significant differences were noted between the U-PASTA treatment and untreated control groups.

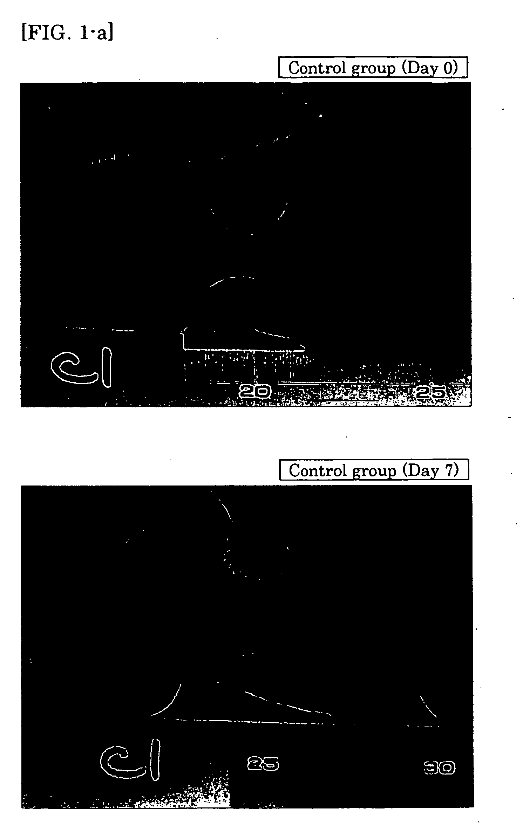

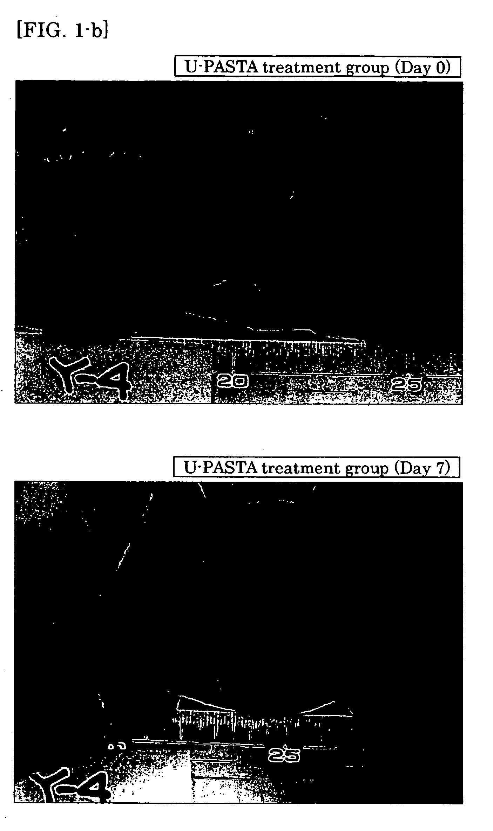

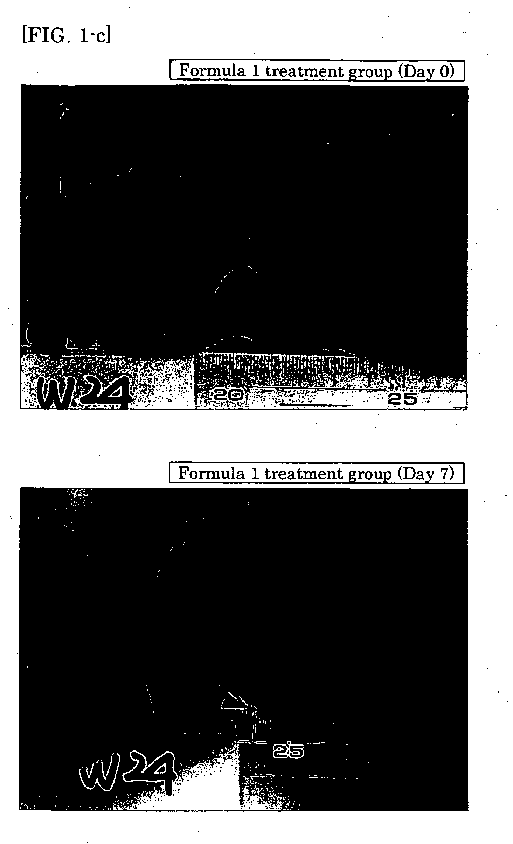

The appearance of the wound healing process is shown in Fig. 1.

In the control (Fig. 1-a) and U-PASTA treatment (Fig. 1-b) groups, very thick scab were formed. Scars on the wound surface and brown dirt showing residual iodine around the wound were noted on one side in the U-PASTA treatment group.

In the Formula 1 treatment group (Fig. 1-c), no thick scab or brown dirt around the wound were noted, showing 'clean' healing of wound surface.

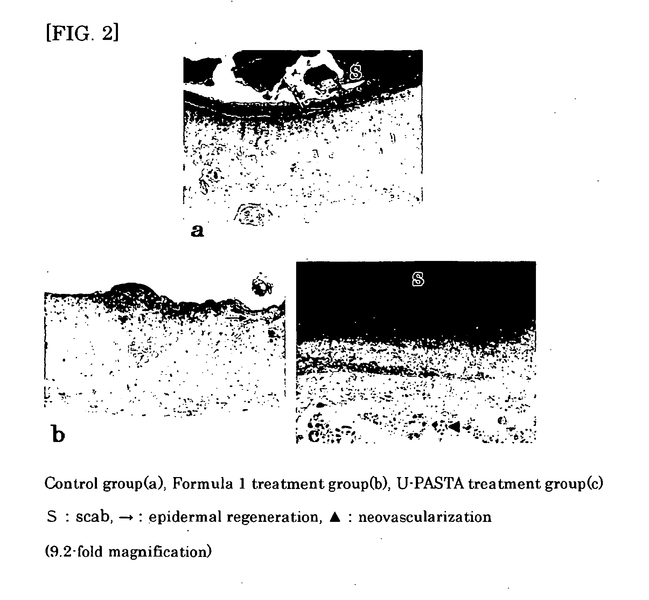

The results of hematoxylin-eosin staining of the wounded skin tissues are shown in Fig. 2.

Granulation was noted in the wounds in all rats in all groups. Especially, in the Formula 1 treatment group, epidermal regeneration was noted in the wound margin (Fig. 2-b).

In a few rats of the U-PASTA treatment group, fibrin aggregates and dead cells remains in the features of the early granulation period, and the features and marked subcutaneous neovascularization were partially noted. (Fig. 2-c).

White soft sugar contained in U-PASTA may have absorbed exudate containing cytokines and growth factors, which reduced fibroblast migration and proliferation, and delayed granulation.
[Table 1]

| Experiment group | Wound area ($cm^2$) | | Healing rate (%) |
|---|---|---|---|
| | Day 0 | Day 7 | |
| Control group (untreated) | 2.49±0.33 | 1.23±0.22 | 49.5±14.9 |
| Formula 1 treatment group | 2.17±0.10 | 0.79±0.18 | 63.6±7.5 |
| U-PASTA treatment group | 2.00±0.29 | 0.97±0.27 | 52.1±8.4 |

The wound area was measured in 5 rats in each group, and presented as the mean ± standard deviation. The healing rate was calculated by the equation below (Equation 1).
[Equation 1]

$$\text{Healing rate (\%)} = 100 \times \{1\text{-(wound area after application for 7 days/ wound area on day 0 of application)}\}$$

[Example 2] Effect on the wound healing process in mice

A wound treatment drug for external use of this invention for skin application (ointments) was prepared using the following formula and method.

[Formula example]

Drugs used for the formula example are shown below.

- White petrolatum (Nacalai Tesque Inc.)
- Isodine gel (Meiji Seika Kaisha Ltd.)
- A commercial disinfectant for disinfection of wound and skin burn surfaces containing 100 mg of povidone iodine and macrogols 4000, macrogols 6000, and macrogols 400 per 1 g.

(Formula 2 (100 g))

White petrolatum: 90.0 g
Isodine gel: 10.0 g
(Production method)

Isodine gel and white petrolatum were mixed at the above ratio and kneaded to a semi-solid state.

<Promotion of wound healing in mice>

[Methods]

Slc:ICR mice (male, 6 weeks of age) were used. Under pentobarbital anesthesia, the dorsal region was shaved, and the skin was punched using a disposable biopsy punch (diameter: 5 mm, Kai Industries Co., Ltd.) to make full-thickness defective wounds.
An appropriate amount of Formula 2 was applied for 7 days.
In a control group, wounds were made and mock treatment was applied to the wounds for 7 days.
The dorsal region was photographed using a digital camera 3 times a week, and the wound areas were measured using imaging analysis software, NIH Image (the National Institutes of Health of the U.S.A.).

The wound area was presented as the mean and standard deviation of 5 mices.

The dorsal skin including the wound surfaces was excised after application for 8 days in each group, and fixed in 10% neutral buffered formalin solution. Paraffin sections were prepared, stained with hematoxylin-eosion, and observed including pathological observation.

[Results]

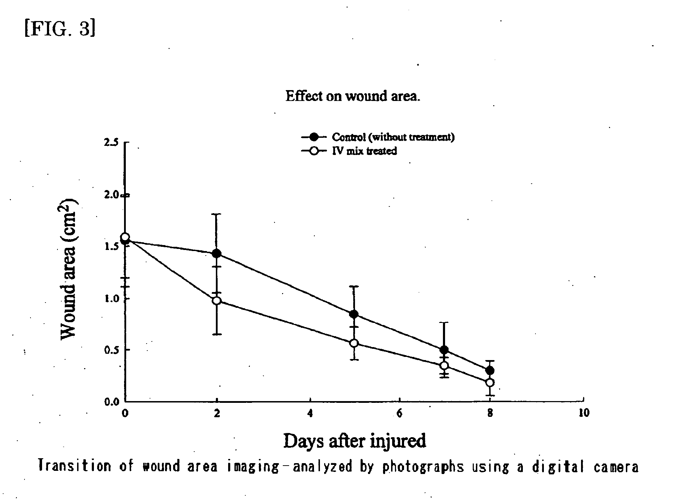

Based on Table 2 <Reduction of wound area and the healing rate> shown below and Fig. 3, the wound area was reduced in both the control and Formula 2 treatment groups, but the healing rate in the Formula 2 treatment group was about 88%, showing faster healing than the control group.

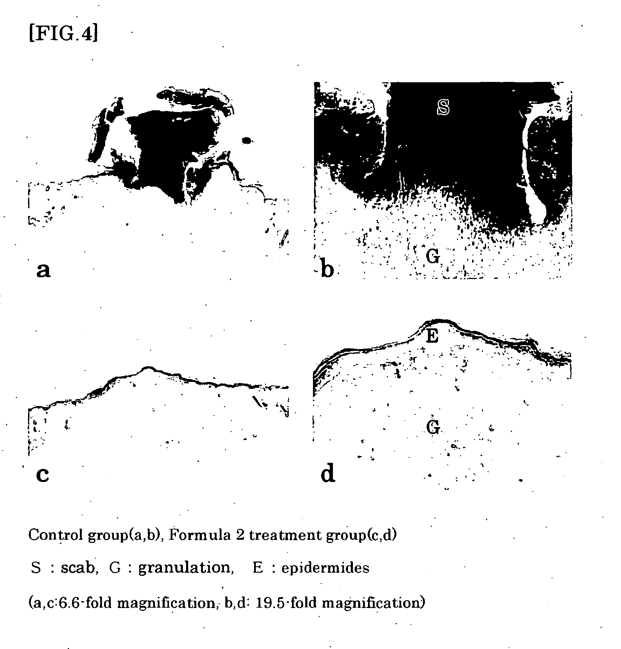

As shown in Fig. 4, granulation was noted in all mices in both the control and Formula 2 treatment groups, but new inflammatory reaction was not noted, suggesting that the injured tissue was in the repair (healing) step.

Epidermal regeneration was noted in all mices in the Formula 2 group, but only 2 of the 5 mices in the control group.

[Table 2]

| Experiment group | Wound area (cm$^2$) | | Healing rate (%) |
|---|---|---|---|
| | Day 0 | Day 7 | |
| Control group (untreated) | 1.55±0.44 | 0.30±0.09 | 79.1±9.5 |
| Formula 2 treatment group | 1.59±0.39 | 0.18±0.12 | 88.4±7.4 |

The wound area was measured in 5 mices in each group, and presented as the mean ± standard deviation. The healing rate was calculated by the equation below (Equation 2).

[Equation 2]

$$\text{Healing rate (\%)} = 100 \times \{1\text{-(wound area after application for 7 days/ wound area on day 0 of application)}\}$$

Fig. 1a-c is photographs showing the therapeutic effect of a drug of this invention on the rat wound surface.

Fig. 2 is photographs of the hematoxylin-eosin-stained rat wound tissues showing the therapeutic effect of a drug of this invention.

Fig. 3 shows the therapeutic effect of a drug of this invention presented as the wound area in mice.

Fig. 4 is photographs of the hematoxylin-eosin-stained mouse wound tissues showing the therapeutic effect of a drug of this invention.

**Claims**

1. A wound treatment drug for topical use which comprises oily base selected from the group consisting of petrolatum and gelated hydrocarbon at a rate of 50-99 weight % and iodine in the form of povidone iodine, and contains substantially no white soft sugar, which is formulated as W/O emulsion.

2. A wound treatment drug for topical use which comprises oily base selected from the group consisting of petrolatum and gelated hydrocarbon at a rate of 50-99 weight % and iodine in the form of povidone iodine, and contains no white soft sugar, which is formulated as W/O emulsion.

3. The drug according to claim 1 or 2, wherein the oily base is petrolatum selected from the group consisting of white petrolatum and hydrophilic petrolatum.

**4.** The drug according to any of claims 1 to 3, wherein the content of said iodine is 0.01-5 weight %.

**5.** The drug according to any of claims 1 to 4, wherein the dosage form of said drug is selected from paste, ointment, cream, liquid, gel, adhesive, cataplasm, and patch.

**6.** The drug according to any of claims 1 to 5, wherein said wound is one selected from the group consisting of erosion, cut, abrasion, burns, decubitus, and skin ulcer.

**Patentansprüche**

**1.** Wundbehandlungs-Arzneimittel zur topischen Verwendung, das eine ölige Grundlage, ausgewählt aus der Gruppe, bestehend aus Petrolat und geliertem Kohlenwasserstoff in einem Anteil von 50 - 99 Gew.-% und Iod in Form von Povidon-Iod, umfasst und im Wesentlichen keinen weißen Farinzucker enthält und als W/O-Emulsion formuliert ist.

**2.** Wundbehandlungs-Arzneimittel zur topischen Verwendung, das eine ölige Grundlage, ausgewählt aus der Gruppe, bestehend aus Petrolat und geliertem Kohlenwasserstoff in einem Anteil von 50 - 99 Gew.-% und Iod in Form von Povidon-Iod, umfasst und keinen weißen Farinzucker enthält und als W/O-Emulsion formuliert ist.

**3.** Arzneimittel nach Anspruch 1 oder 2, wobei die ölige Grundlage Petrolat, ausgewählt aus der Gruppe, bestehend aus weißem Petrolat und hydrophilem Petrolat, ist.

**4.** Arzneimittel nach einem der Ansprüche 1 bis 3, wobei der Gehalt an Iod 0,01 - 5 Gew.-% beträgt.

**5.** Arzneimittel nach einem der Ansprüche 1 bis 4, wobei die Dosierform des Arzneimittels ausgewählt ist aus Paste, Salbe, Creme, Flüssigkeit, Gel, Haftmittel, Kataplasma und Pflaster.

**6.** Arzneimittel nach einem der Ansprüche 1 bis 5, wobei die Wunde ausgewählt ist aus der Gruppe, bestehend aus Erosion, Schnittwunde, Schürfwunde, Verbrennungen, Wundliegen und Hautgeschwür.

**Revendications**

**1.** Médicament pour traiter les plaies à usage topique, qui comprend une base huileuse choisie dans le groupe constitué d'un pétrolatum et d'un hydrocarbure gélifié à un taux de 50 à 99 % en poids et de l'iode sous la forme de polyvidone iodée, et ne contient pratiquement aucun sucre blanc doux, qui est formulé sous la forme d'une émulsion eau/huile.

**2.** Médicament pour traiter les plaies à usage topique, qui comprend une base huileuse choisie dans le groupe constitué d'un pétrolatum et d'un hydrocarbure gélifié à un taux de 50 à 99 % en poids et de l'iode sous la forme de polyvidone iodée, et ne contient pas de sucre blanc doux, qui est formulé sous la forme d'une émulsion eau/huile.

**3.** Médicament selon la revendication 1 ou 2, dans lequel la base huileuse est un pétrolatum choisi dans le groupe constitué d'un pétrolatum blanc et d'un pétrolatum hydrophile.

**4.** Médicament selon l'une quelconque des revendications 1 à 3, dans lequel la teneur en iode est de 0,01 % en poids à 5 % en poids.

**5.** Médicament selon l'une quelconque des revendications 1 à 4, dans lequel la forme galénique dudit médicament est choisie parmi une pâte, une pommade, une crème, un liquide, un gel, un adhésif, un cataplasme et un timbre cutané.

**6.** Médicament selon l'une quelconque des revendications 1 à 5, dans lequel ladite plaie est choisie dans le groupe constitué d'une érosion, d'une coupure, d'une abrasion, d'une brûlure, d'une escarre de décubitus et d'un ulcère cutané.

[FIG. 1-a]

Control group (Day 0)

Control group (Day 7)

[FIG. 1·b]

U·PASTA treatment group (Day 0)

U·PASTA treatment group (Day 7)

[FIG. 1-c]

Formula 1 treatment group (Day 0)

Formula 1 treatment group (Day 7)

[FIG. 2]

Control group(a), Formula 1 treatment group(b), U-PASTA treatment group(c)

S : scab, → : epidermal regeneration, ▲ : neovascularization

(9.2-fold magnification)

[FIG. 3]

Effect on wound area.

—●— Control (without treatment)
—○— IV mix treated

Wound area (cm$^2$)

Days after injured

Transition of wound area imaging-analyzed by photographs using a digital camera

[FIG.4]

Control group(a,b), Formula 2 treatment group(c,d)

S : scab, G : granulation, E : epidermides

(a,c:6.6-fold magnification, b,d: 19.5-fold magnification)

**EP 1 872 788 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1449521 A
- JP 11049672 A
- EP 0185490 A
- JP 10001443 A